(19) Europäisches Patentamt
European Patent Office
Office européen des brevets

(11) Veröffentlichungsnummer: **0 300 309 A1**

(12) **EUROPÄISCHE PATENTANMELDUNG**

(21) Anmeldenummer: **88111032.4**

(51) Int. Cl.⁴: **A61K 6/10**

(22) Anmeldetag: **11.07.88**

(30) Priorität: **22.07.87 DE 3724243**

(43) Veröffentlichungstag der Anmeldung: **25.01.89 Patentblatt 89/04**

(84) Benannte Vertragsstaaten: **AT BE CH DE ES FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Schwabe, Peter, Dr.**
**Dudweiler Strasse 17**
**D-5090 Leverkusen(DE)**
Erfinder: **Voigt, Reiner, D.I.**
**An der Lichtenburg 4**
**D-5090 Leverkusen(DE)**

(54) **Autodesinfizierendes Abformmaterial.**

(57) Abformmaterialien enthalten als Komponente Desinfektionsmittel.

EP 0 300 309 A1

Xerox Copy Centre

## Autodesinfizierendes Abformmaterial

Die Erfindung betrifft Abformmaterialien auf Basis von Hydrokolloiden, bevorzugt irreversible Typen wie z.B. Alginate, die Desinfektionsmittel enthalten.

Bei der Herstellung von Inlays, Kronen, Brücken und Prothesen im Zahnbereich stellt man mit Hilfe von Abformmassen ein Negativ des interessierenden Bereich her, das anschließend mit Modellgips ausgegossen wird. Anhand des Gipsmodells (Positiv) kann man dann die gewünschten zahntechnischen Arbeiten vornehmen.

Als Abformmaterialien werden bevorzugt irreversible Hydrokolloide verwendet. Für Abformungen in der Zahnarztpraxis werden im besonderen Alginate und Agar-Agar eingesetzt.

Abformmaterialien aus irreversiblen Hydrokolloiden werden seit langer Zeit zur Herstellung von Abdrücken im Dentalbereich verwendet (US 23 45 255). Die Verwendung von Agar-Agar in Abformmaterialien ist ebenfalls bekannt (H.J. Rehberg, Die Quintessenz der zahnärztlichen Abformmittel, Verlag "Die Quintessenz" Berlin 1971, S. 18, 57-61).

Hydrokolloide bilden einen Nährboden für pathogene Keime, wie Viren, Bakterien, Pilze und Sporen. Es besteht ein erhebliches Risiko, daß diese Keime von erkrankten Zahnarztpatienten über die Abformungen weiter getragen und verbreitet werden.

Diese Problematik ist bekannt und wird eingehend in Dentalecho 1/87, Seiten 51 bis 54 (1987) behandelt. In der Publikation wird festgestellt, daß noch kein geeignetes Desinfektionsmittel für die Hydrokolloidkategorie gefunden wurde. Praktikable Maßnahmen zur Desinfektion sollen keinen Verlust der Abdruckpräzision zur Folge haben.

Dentalecho 1/87, Seiten 51 bis 54 (1987) sieht eine weitere Schwierigkeit bei der Desinfektion von Abdrücken in Hygienedefiziten in der Zahnarztpraxis. Nach Auffassung der Publikation wird in Zahnarztpraxen das Infektionsrisiko oft falsch eingeschätzt. Dies hängt auch damit zusammen, daß Risikopatienten oft schwierig zu erkennen sind.

Damit sichergestellt ist, daß die Kontaminationskette unterbrochen wird, muß gewährleistet sein, daß die hergestellten Abdrücke in allen Fällen durch Abtötung der pathogenen Keime desinfiziert werden.

Es sind Wirkstoffkombinationen auf Basis von Glutaraldehyd und gepufferten wäßrigen Phenolatlösungen be kannt (US 41 03 001, Firmenschrift Sporicidin der Breitschmidt AG, CH 6020 Kriens), die zur Kaltdesinfektion von Abdrücken aus Alginat verwendet werden können. Der Firmenschrift zur Folge werden zur Desinfektion die Abdrücke kurz in eine 1 zu 16 wäßrige Sporicidinlösung eingetaucht und ohne abzuspülen verpackt.

Diese Methode des Desinfektion hat eine Reihe von Nachteilen. So hängt das Ausmaß der Desinfektion wesentlich von der Zeit des Eintauchens des Abdrucks in die wäßrige Desinfektionslösung ab. Ein zu kurzes Eintauchen in die Desinfektionslösung führt zu einer unvollständigen Abtötung der Keime. Ein zu langes Eintauchen führt auf der anderen Seite zu einer Veränderung an der Oberfläche des Abdrucks und damit zu einer Beeinflussung der Paßform.

Diese bekannte Art der Desinfektion von Abdrücken stellt außerdem nicht sicher, daß die Desinfektion lückenlos in jedem Fall durchgeführt wird. Es wird immer die Unsicherheit verbleiben, ob man nach der Herstellung des Abdrucks eine Desinfektion für erforderlich gehalten hat oder nicht.

Es wurden autodesinfizierende Abformmaterialien auf Hydrokolloidbasis gefunden, die dadurch gekennzeichnet sind, daß sie ein Desinfektionsmittel enthalten.

Die erfindungsgemäßen autodesinfizierenden Abformmaterialien gewährleisten eine Abtötung der pathogenen Keime auf dem Material nach einer Kontamination. Eine Veränderung des Paßgenauigkeit des Abdrucks durch eine nachfolgende Sterilisation entfällt. Außerdem entfällt die Unsicherheit, ob eine Desinfektion durchgeführt wurde. Die erfindungsgemäßen autodesinfizierenden Abformmaterialien stellen eine Unterbrechung der Kontaminationskette sicher.

Außerdem weisen die erfindungsgemäßen pulverförmigen Abformmaterialien überraschenderweise eine erhöhte Lagerstabilität auf. Auch nach langer Lagerung bleiben die hohen Qualitätsmerkmale der Abformungen erhalten.

Erfindungsgemäße Desinfektionsmittel sind bevorzugt Phenol-Derivate und Ammoniumsalze.

Als Phenol-Derivate seien Verbindungen wie Kresole, Chlorkresole, Xylole, Chlorxylol, Isopropylkresol, Chlorthymol, Phenylphenole und Bis-Phenole genannt.

Als Ammoniumsalze seien Verbindungen der Formel

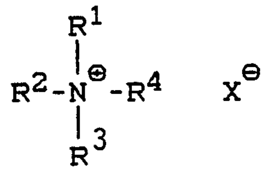

in der
$R^1$, $R^2$, $R^3$ und $R^4$ gleich oder verschieden sind und Methyl, Ethyl, Propyl, Butyl, Pentyl, Hexyl, Heptyl, Octyl, Nonyl, Decyl, Undecyl, Dodecyl, Tetradecyl, Hexadecyl, Octadecyl, Pyridinyl und Benzyl, wobei $R^1$ und $R^2$ auch zu einem Pyridylring verbunden sein können,
bedeuten und
X für Chlor und Brom steht,
genannt.

Beispielsweise seien die folgenden Phenol-Derivate genannt:
2-Isopropyl-5-methyl-phenol,
4-Chlor-2-methyl-phenol,
4-Chlor-3,5-dimethyl-phenol,
4-Chlor-2-isopropyl-5-methyl-phenol,
4-Chlor-3-isopropyl-6-methylphenol,
2,2′-Methylen-di-(4-chlorphenol),
2-Phenol-phenol,
2-Phenyl-phenol-Na-salz.

Beispielsweise seien die folgenden Ammoniumsalze genannt:
Hexadecyl-pyridiniumchlorid,
Hexadecyl-trimethylammoniumbromid,
Hexadecyl-trimethylammoniumchlorid,
Benzyl-dodecyl-dimethyl-ammoniumchlorid.

Bevorzugte erfindungsgemäß eingesetzte Desinfektionsmittel sind
2-Isopropyl-5-methyl-phenol,
4-Chlor-2-methyl-phenol,
4-Chlor-3,5-dimethyl-phenol,
2,2′-Methylen-bis(4-chlorphenol),
2-Phenylphenol und dessen Na-Salz,
Hexadecylpyridiniumchlorid.

Insbesondere bevorzugt wird 2,2′-Methylen-bis-(4-chlorphenol).

Die bevorzugt erfindungsgemäß eingesetzten Desinfektionsmittel sind Feststoffe und liegen als Bestandteil des Abformmaterials aus dem Hydrokolloid vor.

Die erfindungsgemäßen Desinfektionsmittel liegen im allgemeinen pulverförmig vor.

Die erfindungsgemäß eingesetzten Desinfektionsmittel haben im allgemeinen eine Korngröße von 1 bis 100 µm, bevorzugt von 5 bis 40 µm.

Die erfindungsgemäßen Abformmaterialien enthalten die Desinfektionsmittel in einer Menge von 0,05 bis 1,0 Gew.-Teilen, bevorzugt von 0,1 bis 0,5 Gew.-Teilen, bezogen auf 100 Gew.-Teile der Zubereitung.

Die Hydrokolloide können irreversible und reversibel sein. Bevorzugt werden Alginate und Agar-Agar.

Im allgemeinen enthalten die erfindungsgemäßen Abformmaterialien ein lösliches Hydrokolloid (z.B. das Natrium und/oder Kaliumsalz der Alginsäure) in einer Menge von vorzugsweise 8 bis 25 Gew.-%, insbesondere 10 bis 17 Gew.-%, eine Metallverbindung, die mit Alginsäure ein wasserunlösliches Salz bildet (vorzugsweise Calcium-verbindungen wie Calciumsulfat) in einer Menge von vorzugsweise 5 bis 40 Gew.-%, insbesondere von 10 bis 25 Gew.-%, ein Verzögerer für die Aushärtung (z.B. Alkaliphosphat, -diphosphat oder -polyphosphat) in einer Menge von vorzugsweise 0,5 bis 10 Gew.-%, insbesondere 1 bis 5 Gew.-%, sowie Füllstoffe (z.B. Gips, Kieselerde, Diatomeenerde oder Ton) in einer Menge von vorzugsweise 25 bis 85 Gew.-%, insbesondere 40 bis 75 Gew.-%, Gegebenenfalls können weitere Zuschlagstoffe wie z.B. Farb-und Geschmackstoffe, sowie die Verträglichkeit mit Gips verbessernde Verbindungen (z.B. Kaliumfluortitanat oder Kaliumfluorzirkonat) in der Abformmasse enthalten sein. Weitere Additive, die z.B. das Stauben des Materials verhindern, sind selbstverständlich auch möglich.

Es wurde auch ein Verfahren zur Herstellung von autodesinfizierenden Abformmaterialien gefunden, das dadurch gekennzeichnet ist, daß man das pulverförmige Desinfektionsmittel mit der 1 bis 50 fachen, vorzugsweise 10 bis 20 fachen Menge Füllstoffe vormischt und über ein Sieb mit 150 bis 400 µm Maschenweite in einem Mischer zu den anderen Einsatzstoffen gibt und vermischt.

Im allgemeinen verwendet man Siebe mit 150 bis 400, bevorzugt 200 bis 300 µm Maschenweite.

Als Mischer werden bevorzugt Pflugscharmischer eingesetzt.

Die erfindungsgemäßen autodesinfizierenden Abformmaterialien werden in üblicher Weise für die Anwendung zubereitet. Man rührt das pulverförmige Material in der erforderlichen Menge Wasser an und appliziert es für den Abdruck in den entsprechenden Zahnbereich.

Die erfindungsgemäßen autodesinfizierenden Abformmaterialien weisen ein weites Wirkstoffspektrum auf und erfassen praktisch alle pathogenen Keime. Als pathogene Keime seien alle bekannten Viren, Bakterien, Pilze und Sporen genannt. Beispielsweise seien die folgenden pathogenen Keime genannt:
Staphylococcus aureus,
Escherichia coli
Candida albicans.

Die erfindungsgemäßen autodesinfizierenden Abformmaterialien weisen hervorragende Abformeigenschaften auf.

Beispiel 1 (Herstellung einer Alginatabformmasse)

In einem Pflugscharmischer werden
58,00 Gew.-Teile Diatomeenerde,
8,00 Gew.-Teile Aluminiumsilikat,
15,45 Gew.-Teile Calciumdihydrat,
14,00 Gew.-Teile Natriumalginat,
3,00 Gew.-Teile Kaliumfluortitanat,
1,20 Gew.-Teile Tetranatriumdiphosphat,
0,30 Gew.-Teile Farbpigment und
0,05 Gew.-Teile Duftstoff
zu einer homogenen Mischung verarbeitet.

Die Desinfektionsmittel werden in einer Kugelmühle bis zu einer Korngröße von < 40 µm gemahlen. Die gepulverten Desinfektionsmittel werden in einer Menge von 0,1 bzw. 0,3 Gew.-Teilen mit 2 bzw. 5 Gew.-Teilen von obiger Mischung vorgemischt, durch ein Sieb mit 200 µm Maschenweite gegeben und in Pflugscharmischer mit den übrigen 98 bzw. 95 Gew.-Teilen obiger Mischung vermischt.

Beispiel 2 bis 14

a) Die mikrobiologische Wirksamkeit der Desinfektionsmittel in dem Abformmaterial nach Beispiel 1 wird wie folgt bestimmt:
In Anmischbechern werden die Komponenten vorgelegt und mit dem Anmischspatel innerhalb von 30 Sekunden vermischt. Es werden Probestücke von 6 cm Durchmesser und 0,5 cm Höhe hergestellt. Aus diesen Scheiben werden Prüfstücke von 3 cm ausgestanzt.
Petrischalen von 9,5 cm Durchmesser werden mit 10 ml kontaminiertem Agar gefüllt. Die Keimsaat beträgt 1 x $10^4$ Keime/l ml Agar. Nach dem Erstarren des Nährbodens werden die Prüfkörper mittig auf den Nährboden aufgelegt und leicht aufgedrückt, damit ein intensiver Kontakt zu dem Nährboden entsteht.
Die Lagerung der Prüfkörper erfolgt bei 27° C über einen Zeitraum von 4 Tagen.

b) In der Tabelle 1 sind die Prüfungsergebnisse aufgeführt. Außer den mikrobiologischen Daten enthält die Tabelle Ergebnisse der Prüfung der Alginat-Abformmassen nach der Spezifikation Nr. 18 der American Dental Association (ADA).

Tabelle 1

| Bsp. | Desinfektionsmittel | Konzentration in Gew.-% | mikrobiologische Beurteilung | Prüfung nach ADA-Spezifikation Nr. 18 | | |
|---|---|---|---|---|---|---|
| | | | | Abbindezeit | bleib. Deform. (%) | Druckfest. ($N/mm^2$) |
| 2 | ohne Wirkstoff | - | 0 | 1'10" | 2,5 | 0,90 |
| 3 | 2,2'Methylen-bis-(4-chlorphenol) | 0,1 | 5-7 | 1'25" | 2,5 | 0,98 |
| 4 | 2,2'-Methylen-bis-(4-chlorphenol) | 0,3 | 6-9 | 1'30" | 3,4 | 0,85 |
| 5 | 2-Phenylphenol | 0,1 | 2-3 | 1'10" | 2,5 | 0,92 |
| 6 | 2-Phenylphenol | 0,3 | 4-5 | 1'10" | 2,5 | 0,88 |
| 7 | 4-Chlor-2-methylphenol | 0,1 | 1-1,5 | 1'15" | 2,5 | 0,89 |
| 8 | 4-Chlor-2-methylphenol | 0,3 | 4-5 | 1'20" | 2,5 | 0,90 |
| 9 | 2-Isopropyl-5-methylphenol | 0,1 | 0 | 1'15" | 2,1 | 0,92 |
| 10 | 2-Isopropyl-5-methylphenol | 0,3 | 2-3,5 | 1'20" | 2,2 | 0,86 |
| 11 | 2-Phenylphenol, Na-Salz | 0,1 | 1 | 1'25" | 3,0 | 0,92 |
| 12 | 2-Phenylphenol, Na-Salz | 0,3 | 3-4 | 1'30" | 2,6 | 1,00 |
| 13 | Hexadecylpyridiniumchlorid | 0,1 | 0 | 1'10" | 2,9 | 0,95 |
| 14 | Hexadecylpyridiniumchlorid | 0,3 | 1-2 | 1'15" | 3,5 | 0,95 |

Die Werte in der mikrobiologischen Beurteilung stellen den Hemmhof um den Alginatprobekörper in der kontaminierten Agarmasse in mm dar.

Zur Vertiefung der Ergebnisse aus Tabelle 1 wurden die Alginat-Abformmassen unbehandelt (Beispiel 2 als Vergleich), mit 0,1 und 0,3 Gew.-% 2,2′-Methylen-bis-(4-chlorphenol) (Beispiele 3 und 4) bzw. mit 0,1 und 0,3 Gew.-% 2-Phenylphenol versetzt (Beispiele 5 und 6) nochmals nach 3 Monaten und 6 Monaten, 1 und 2 Jahren, Lagerung der Spezifikationsprüfung nach ADA 18 unterworfen; es zeigt sich, daß die Lagerstabilität praktisch unverändert bleibt. Darüber hinaus wurde nach der beschriebenen Methode die mikrobiologische Wirksamkeit der beiden Desinfektionsmittel gegenüber den Prüfkeimen Staphylococcus aureus, Escherichia coli und Candida albicans festgestellt.

Die Ergebnisse der Prüfungen sind in den Tabellen 2 und 3 zusammengefaßt.

Tabelle 2

| | | Konzentration Gew.-% | Wirksamkeit gegen | | |
|---|---|---|---|---|---|
| | | | Escherichia coli | Staphylococcus aureus | Candida albicans |
| 2 | ohne Wirkstoff (Vergleich) | - | - | - | - |
| 3 | 2,2′-methylene-bis-(4-chlorophenol) | 0,1 | 5 | 10 | 7 |
| 4 | 2,2′-methylen-bis-(4-chlorophenol) | 0,3 | 11 | 13 | 10 |
| 5 | 2-phenylphenol | 0,1 | 2,5 | 1,5 | 3 |
| 6 | 2-phenylphenol | 0,3 | 5 | 7 | 13 |

Tabelle 3

| | | Gew.-% | Test nach AOA No 18 | | | | | | | | | | | | | | |
|---|---|---|---|---|---|---|---|---|---|---|---|---|---|---|---|---|---|
| | | | Abbindezeit | | | | | bleibende Deformation | | | | | Druckfestigkeit (N/mm²) | | | | |
| | | | a | b | c | d | e | a | b | c | d | c | a | b | c | d | e |
| 2 | ohne Wirkstoff | - | 1'10" | 1'30" | 1'45" | 1'55" | 2'10" | 2,5 | - | 2,5 | 3,3 | 5,2 | 0,90 | - | 0,83 | 0,79 | 0,53 |
| 3 | 2,2'-Methylene-bis-(4-chlorophenol) | 0,1 | 1'25" | 1'35" | 1'35" | 1'40" | 1'40" | 2,5 | - | 2,5 | 2,8 | 2,9 | 0,98 | - | 0,86 | 0,82 | 0,76 |
| 4 | 2,2'-Methylene-bis-(4-chlorophenol) | 0,3 | 1'30" | 1'35" | 1'35" | 1'35" | 1'40" | 3,4 | - | 2,5 | 2,5 | 2,9 | 0,85 | - | 0,82 | 0,83 | 0,78 |
| 5 | 2-Phenylphenol | 0,1 | 1'10" | 1'25" | 1'30" | 1'35" | 1'45" | 2,5 | - | 2,6 | 3,0 | 3,3 | 0,92 | - | 0,85 | 0,80 | 0,73 |
| 6 | 2-Phenylphenol | 0,3 | 1'10" | 1,25" | 1'30" | 1'30" | 1'40" | 2,5 | - | 2,6 | 3,0 | 3,2 | 0,88 | - | 0,83 | 0,78 | 0,78 |

a = sofort

b = nach 3 Monaten

c = nach 6 Monaten

d = nach 1 Jahr

nach 2 Jahren

## Ansprüche

1. Autodesinfizierende Abformmaterialien auf Hydrokolloidbasis, dadurch gekennzeichnet, daß sie Desinfektionsmittel enthalten.

2. Autodesinfizierende Abformmaterialien auf Hydrokolloidbasis nach Anspruch 1, dadurch gekennzeichnet, daß sie pulverförmige Desinfektionsmittel enthalten.

3. Autodesinfizierendes Abformmaterial nach den Ansprüchen 1 und 2, dadurch gekennzeichnet, daß das Desinfektionsmittel eine Korngröße von 1 bis 100 µm aufweist.

4. Autodesinfizierende Abformmaterialien nach den Ansprüchen 1 bis 3, dadurch gekennzeichnet, daß sie als Desinfektionsmittel Phenol-Derivate, Halogenphenol-Derivate und/oder ein Phenylphenol oder dessen Salze enthalten.

5. Autodesinfizierende Abformmaterialien nach den Ansprüchen 1 bis 3, dadurch gekennzeichnet, daß sie als Desinfektionsmittel quarternäre Ammoniumverbindungen enthalten.

6. Autodesinfizierendes Abformmaterial nach den Ansprüchen 1 bis 5, enthaltend 0,05 bis 1,0 Gew.-Teile Desinfektionsmittel bezogen auf 100 Gew.-Teile des Abformmaterials.

7. Autodesinfizierendes Abformmaterial nach den Ansprüchen 1 bis 6, dadurch gekennzeichnet, daß es ein Hydrokolloid, ein Geliermittel, ein Verzögerer, Füll- und Zuschlagstoffe, ein Desinfektionsmittel und gegebenenfalls weitere Additive enthält.

8. Verfahren zur Herstellung von autodesinfizierenden Abformmaterialien, dadurch gekennzeichnet, daß das pulverförmige Desinfektionsmittel mit der 1- bis 50-fachen Menge Füllstoff vorgemischt und über ein Sieb mit 150 bis 400 µm Maschenweite in einen Mischer zu den anderen Einsatzstoffen gegeben und vermischt wird.

9. Verwendung von autodesinfizierenden Abformmaterialien enthaltend ein Desinfektionsmittel zur Herstellung von Abdrücken im Dentalbereich.

Europäisches Patentamt

# EUROPÄISCHER RECHERCHENBERICHT

Nummer der Anmeldung

EP 88 11 1032

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| E | EP-A-0 265 776 (DENTSPLY)<br>* Insgesamt * | 1-9 | A 61 K 6/10 |
| X | US-A-4 060 421 (I. YOSHIKAWA)<br>* Spalte 4, Zeile 44 - Spalte 5, Zeile 14 * | 1,9 | |
| X | US-A-3 850 864 (D. EMERSON)<br>* Spalte 3, Zeilen 19-29; Spalte 4, Zeilen 3-22 * | 1,5,9 | |
| Y | | 1-9 | |
| Y | DE-A-1 494 347 (STERIL-PLAST)<br>* Ansprüche * | 1,4 | |
| Y | EP-A-0 141 628 (UNITIKA)<br>* Ansprüche * | 1,5 | |
| A | R. PHILLIPS: "Skinner's Science of Dental Materials", 8. Ausgabe, Seiten 126-128, W.B. SAUNDERS CO., Philadelphia, US | 1-9 | |

RECHERCHIERTE SACHGEBIETE (Int. Cl.4)

A 61 K
C 08 L
C 08 K

Der vorliegende Recherchenbericht wurde für alle Patentansprüche erstellt

| Recherchenort | Abschlußdatum der Recherche | Prüfer |
|---|---|---|
| DEN HAAG | 04-10-1988 | COUSINS-VAN STEEN G.I.L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)